# EUROPEAN PATENT APPLICATION

(11) **EP 4 218 576 A1**
(43) Date of publication of application: **02.08.2023**
(21) Application number: 21873994.4
(22) Date of filing: 20.07.2021
(51) Int. Cl.: A61B 5/16

(54) **SLEEP MONITORING METHOD AND APPARATUS, ELECTRONIC DEVICE, AND COMPUTER-READABLE MEDIUM**

(30) Priority: 29.09.2020 CN 202011052065
(71) Applicant: GUANGDONG OPPO MOBILE TELECOMMUNICATIONS CORP., LTD., Dongguan, Guangdong 523860 (CN)
(72) Inventor: YAN, Haizhou, Dongguan, Guangdong 523860 (CN)
(74) Representative: Novagraaf Technologies
(86) International application number: PCT/CN2021/107404
(87) International publication number: WO 2022/068332

(57) **Abstract**

Disclosed are a method and apparatus for sleep monitoring, an electronic device and a computer-readable medium, relating to the field of health monitoring technology, the method comprises: a screen state of a target terminal is obtained; physiological parameters of a user are obtained, the physiological parameters including at least one of body motion parameters and heart rate parameters; a sleep state of the user is determined based on the screen state and the physiological parameters. Since the screen state can reflect the user's operation of the user terminal, and the operation can further reflect the sleep state of the user, and the physiological parameters can also reflect the sleep state of the user, the combination of the screen state and the physiological parameters can make the determination of sleep state more accurate.

## Description

### CROSS-REFERENCE TO RELATED APPLICATIONS

This application claims priority to Chinese patent application No. 202011052065.2, entitled "Method and Apparatus for Sleep Monitoring, Electronic Device and Computer-readable Medium", filed with the China Patent Office on September 29, 2020, the entire contents of which are incorporated by reference in this application.

### TECHNICAL FIELD

The present application relates to the field of health monitoring technologies, and more specifically, to a method and apparatus for sleep monitoring, an electronic device, and a computer-readable medium.

### BACKGROUND

With the popularity of wearable devices or mobile terminals such as smart phones, smart watches, smart bracelets (abbreviated as: "bracelets"), the sleep health monitoring technology based on smart devices has gradually matured. However, it is not high for the current accuracy of sleep monitoring, especially for users that play with cell phones in bed, the error of sleep monitoring will be greater.

### SUMMARY

A method and apparatus for sleep monitoring, an electronic device, and a computer-readable medium are provided in the embodiments of the present disclosure to improve above mentioned defects.

In a first aspect, an embodiment of the present application provides a method for sleep monitoring, applied to an electronic device, the method including: a screen state of a target terminal is acquired; physiological parameters of a user are acquired, the physiological parameters including at least one of body motion parameters and heart rate parameters; a sleep state of the user is determined based on the screen state and the physiological parameters, the sleep state including a sleeping state and an awake state.

In a second aspect, an embodiment of the present application further provides an apparatus for sleep monitoring including a first acquisition unit, a second acquisition unit and a determination unit. The first acquisition unit, configured to acquire the screen state of a target terminal. The second acquisition unit, configured to acquire physiological parameters of a user, the physiological parameters including at least one of body motion parameters and heart rate parameters. The determination unit, configured to determine a sleep state of the user based on the screen state and the physiological parameters, the sleep state including a sleeping state and an awake state.

In a third aspect, an embodiment of the present application further provides an electronic device comprising: one or more processors; a memory; one or more applications, wherein the one or more applications are stored in the memory and configured to be executed by the one or more processors, the one or more applications being configured to perform above mentioned method.

In a fourth aspect, an embodiment of the present application further provides a computer-readable medium, the computer-readable medium storing program code executable by a processor, the program code causing the processor to execute above mentioned method when executed by the processor.

The method and apparatus for sleep monitoring, the electronic device and the computer-readable medium provided in the present application obtains the screen state of the target terminal and the physiological parameters of the user, then, determines the sleep state of the user based on the screen state and the physiological parameters, wherein the physiological parameters can be at least one of body motion parameters and heart rate parameters, finally, the sleep state of the user may be determined based on the screen state and physiological parameters.

### BRIEF DESCRIPTION OF DRAWINGS

In order to describe technical solutions in the embodiments of the present application more clearly, drawings that need to be used in the description of the embodiments will be briefly introduced below, it is apparent that the drawings described below are only some embodiments of the present application, and for those of ordinary skills in the art, other drawings may be obtained according to these drawings without paying any inventive effort.
FIG. 1 illustrates a curve of variation of acceleration values in a sleep state provided by an embodiment of the present application.
FIG. 2 illustrates a curve of variation of heart rate in a sleep state provided by an embodiment of the present application.
FIG. 3 illustrates a method flowchart of a method for sleep monitoring provided by an embodiment of the present application.
FIG. 4 illustrates a method flowchart of a method for sleep monitoring provided by another embodiment of the present application.
FIG. 5 illustrates a diagram of a sleep period determined based on acceleration values provided by an embodiment of the present application.
FIG. 6 illustrates a diagram of a sleep period determined based on heart rate provided by an embodiment of the present application.
FIG. 7 illustrates a diagram of a sleep period determined based on screen state provided by an embodiment of the present application.
FIG. 8 illustrates a method flowchart of a method for sleep monitoring provided by another embodiment of the present application.
FIG. 9 illustrates a diagram of a sleep period determined based on HRV provided by an embodiment of the present application.
FIG. 10 illustrates a modular block diagram of an apparatus for sleep monitoring provided by an embodiment of the present application.
FIG. 11 illustrates a modular block diagram of an electronic device provided by an embodiment of the present application.
FIG. 12 illustrates a storage unit provided by an embodiment of the present application configured to save or carry program code implementing an information processing method according to an embodiment of the present application.

### DETAILED DESCRIPTION

For a better understanding of the present application scheme by those in the art, the following is a clear and complete description of the technical solutions in embodiments of the present application in conjunction with the drawings in the embodiments of the present application.

With the accelerated pace of life and increased work pressure, sleep disorders have begun to become a major problem for modern people, with almost more than 60% of people having sleep disorders. There are numerous types of sleep disorders, which are summarized into three main categories: difficulties falling asleep or staying asleep (insomnia), poor quality sleep and sleep deprivation. Sleep disorders can bring a lot of inconvenience to our lives, and will be more likely to lead to accelerated aging, immune system decline, induce cardiovascular disease. As you can see, it is very important to maintain a healthy sleep.

The concept of preventive medicine is currently used to address sleep disorders, i.e., early detection and early treatment. The traditional method of assessing sleep disorders is to fill out a sleep assessment form or to do a full night of PSG monitoring. Although these two methods are quite accurate, it is not practical to do long-term monitoring considering the cost and inconvenience. Therefore, there is an urgent need for a convenient and inexpensive sleep monitoring system to help us identify sleep disorders.

Due to the rapid development of wearable devices and smartphones, more and more sleep monitoring systems have been developed, which can be broadly classified into two categories: acceleration signal-based, heartbeat signal-based, and based on the joint detection of acceleration signal and heartbeat signal.

The inventor found in his research that there are many shortcomings in current methods for sleep monitoring, specifically, although the above mentioned method can monitor sleep state of a user quite accurately in some scenes such as the user lying in bed to sleep, but in some special scenes, for example, the user lying in bed to play cell phones or watch videos and other scenes, the scenes are characterized by the user remains awake but the movement is almost small, therefore, in this special scenes, the monitoring result obtained by using the aforementioned acceleration signal based, heartbeat signal based, and based on the joint detection of acceleration signal and heartbeat signal are usually that the user is asleep, in other word, there is some mistake.

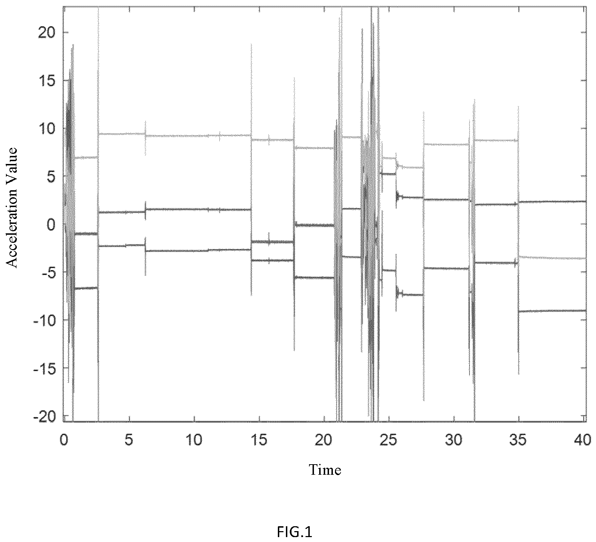

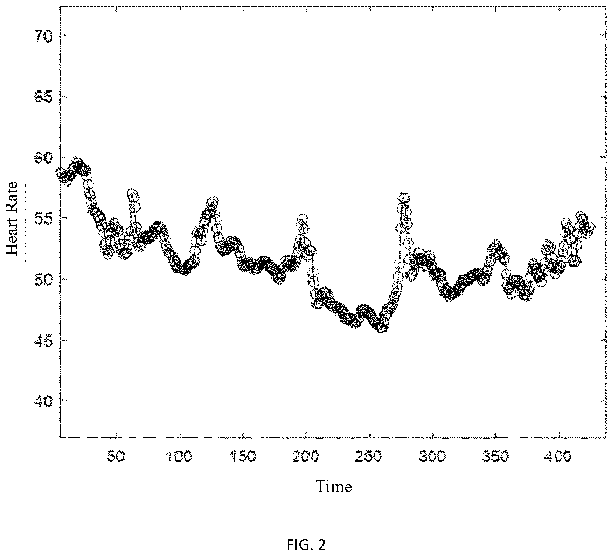

For example, a user is lying in bed watching a video or reading an article on a cell phone, an acceleration signal of the user's body which is obtained by detection is illustrated in FIG. 1, and a heart rate result which is detected is illustrated in FIG. 2. It can be seen that the acceleration value in FIG. 1 shows that the user has very little body movement at this time, and the body is almost in a stationary state. As can be seen from the heart rate values in FIG. 2, the heart rate of the user is quite low and smooth at this time, and the physical characteristics of the user while sleeping are that the body is quite stationary, with less body movement and a lower heart rate, i.e., stable at a resting heart rate. Therefore, it can be seen from FIG. 1 and FIG. 2 that the sleep monitoring results obtained by acceleration and/or heart rate at this time are asleep, however, the state of the user at this time is lying in bed and watching videos on a cell phone. Therefore, the sleep monitoring results do not match with the actual sleep state of the user, i.e., the monitoring result is wrong.

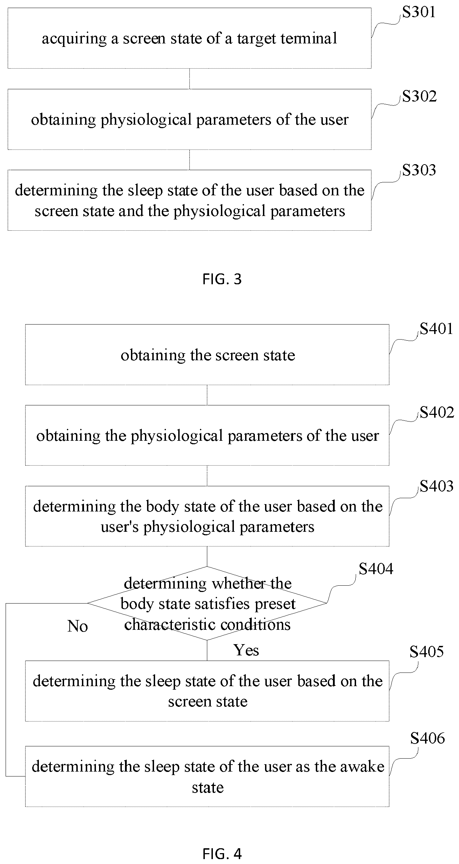

Therefore, in order to improve the above defects, as illustrated in FIG. 3, an embodiment of the present application provides a method for sleep monitoring, which may be applied to a mobile terminal, or to a user's wearable device, or to a server. Wherein, the wearable device may be a bracelet or a smart watch, etc., and the mobile terminal may be a smartphone used by the user. That is to say, the execution subject of the method may be a mobile terminal, a wearable device, an AV playback device, or a server, wherein the server can obtain data from the mobile terminal and the wearable device. Then if the execution subject of the method is a mobile terminal, then the mobile terminal can establish a connection with the wearable device directly and obtain data from the wearable device through the established connection. If the subject of the method is a wearable device, it is also possible to obtain data from the mobile terminal through an established connection. Of course, a mobile terminal and wearable device can also obtain data from a server, that is, one of the two transmits data to the server, and the server forwards the data to the other one. Specifically, the execution subject of the method of the embodiment of the present application may be an electronic device, which may be at least one of the aforementioned mobile terminal, wearable device and server. And then the method includes: S301 to S303.

S301: acquiring a screen state of a target terminal.

Wherein, the target terminal may be at least one of the above-mentioned mobile terminal, wearable device and AV playback device. The screen state may comprise a light-on/light-off state of the screen, an unlocked/locked state of the screen, a touch state, a display state and so on.

Wherein, the light-on/light-off state of the screen may include a screen-light-on state and a screen-light-off state. The screen displays a bright screen in the screen-light-on state, i.e., the backlight panel of the screen is illuminated, and the screen displays a dark screen in the screen-light-off state, i.e., the backlight panel of the screen is not illuminated or the illumination is weak.

As an implementation, the screen state may be the screen state of the mobile terminal, or the screen state of the wearable device, or the AV playback device within the user's current environment, and as an implementation, the AV playback device may be a screened device. Specifically, the screened device may be a device with a screen installed, wherein the user's current environment may be determined based on the user's location. As an implementation, the location information of the user is determined by the mobile terminal or the wearable device of the user, the environment in which the user is currently located is determined based on the location information of the user, and then, the screened device within the environment in which the user is currently located is predetermined. The screened device in the home device corresponding to the user's home address may be counted as the screened device corresponding to the home address, after obtaining the location information of the user, if the location information indicates the home address, the screened device within the user's current environment may be determined as the screened device in the user's home device, for example, a TV set, etc. As another implementation, it may also be that the user is configured with a screened device for the home address. For example, although there is a plurality of screened devices in the user's home address, the user may select at least one of the screened devices as the screened device corresponding to the user's home address. Further, it may be that when the user's location information is determined to be located at the user's home address, the user's location information is further determined to be located in a specific room at the home address based on indoor positioning technology, which is noted as a target room, and the screened device set in the target room is selected as the screened device in the user's current environment.

As an implementation, the target terminal may be a terminal associated with the user, specifically, the identity information corresponding to the user account logged into the target terminal is the identity information of the user. For example, the ID of the real-name authentication of the user account is the same as the ID of the user. In some embodiments, when the user corresponds to multiple terminal devices, the target terminal may be determined by the user's usage data for each terminal device. Specifically, the usage data includes the user's operation of the terminal device, and the recently used terminal device is found from the plurality of terminal devices by the usage data of each terminal device as the target terminal, thus, the screen state of the target terminal is obtained. In other embodiments, if the use location of the target terminal matches the resident address of the user, the target terminal is characterized as associated with the user, wherein the use location is the location where the terminal is located when it is used, and the resident address may include the location where the user frequently resides such as an office address or a home address. For example, the target terminal may be the above-mentioned AV playback device, then the installation location of the AV playback device is located within the specified range of the target address of the user, then the AV playback device is associated with the user. Wherein, the target address may be the user's home address, and the specified range may be set according to actual needs. For example, the AV playback device is a screened device as described above, then if the AV playback device is a screened device among the user's smart home device, the AV playback device is characterized as associated with the user. Alternatively, then the wearable device is a device associated with the user's mobile terminal, specifically, reference may be made to subsequent embodiments.

As an implementation, if the screen state is a screen state of a mobile terminal or an AV playback device, the screen-light-on state and the screen-light-off state of the screen can be detected by a program module within the system of the mobile terminal or the AV playback device. For example, the screen-light-off state and the screen-light-on state are detected by the isScreenOn function of a PowerManager. As another implementation, the screen-light-on state and the screen-light-off state of the mobile terminal or AV playback device can also be collected by the user's wearable device, specifically, the wearable device is equipped with a light sensor that can detect the light intensity of the surrounding environment, and if the user is using the mobile terminal while also wearing the wearable device, or, the user is wearing the wearable device with the wearable device placed around the mobile terminal or AV playback device. Since the screen of the mobile terminal or the AV playback device emits different light intensity in the screen-light-on state and the screen-light-off state, the screen-light-on state and the screen-light-off state of the screen can be detected through the collection of the light intensity of the screen of the mobile terminal or the AV playback device by the wearable device.

Wherein, the unlocked/locked state of a screen includes a locked state and an unlocked state, as an implementation, the broadcasts Intent.ACTION_SCREEN ON and Intent.ACTION_SCREEN_OFF are received by the BroadcastReceiver, so that it can determine whether the mobile terminal is in the locked state or the unlocked state.

Wherein, the touch state of a screen can include a touched state and an untouched state. The touched state refers to the screen has been touched within a specified length of time, and the untouched state refers to the screen has been touched within a specified length of time. As an implementation, it could detect whether the screen is touched at the current moment, if it is touched, then the touch state of the screen is determined to be touched state, otherwise, the touch state of the screen is determined to be untouched state.

As another implementation, it can also detect whether the screen receives a touch operation within a specified length of time before the current time, and determine the touch state of the screen based on the touch operation received by the screen detected within the specified length of time. In some embodiments, if the screen has received a touch operation within the specified length of time, that is, the screen has been touched, the touch state of the screen may be regarded as the touched state, and if the screen has not received a touch operation within the specified length of time, the touch state of the screen may be regarded as the untouched state. In other embodiments, it obtains the number of touch operations received by the screen detected in the specified length of time, if the number is greater than the second threshold, the touch state of the screen is determined to be the touched state, otherwise the number is less than or equal to the second threshold, the touch state of the screen is determined to be the untouched state.

Wherein, the display state of the screen may include a desktop interface state and a non-desktop interface state, wherein the desktop interface state is used to indicate that the content currently displayed on the screen is a system desktop, which displays icons of the applications installed by the target as an operating interface for the user to select and operate the applications from a plurality of applications, and the non-desktop interface state is used to indicate that the content currently displayed on the screen is non-system desktop. Wherein, the non-desktop interface state can also determine a plurality of sub-states according to the type of content displayed, for example, a video interface sub-state, a game interface sub-state, etc., wherein the video interface sub-state is used to indicate that the interface currently displayed on the screen is a video interface, i.e., the content displayed on the screen is a video, and the game interface sub-state is used to indicate that the interface currently displayed on the screen is a game interface, i.e., the content displayed on the screen is the interface of a game application.

S302: obtaining physiological parameters of the user.

wherein the physiological parameters include at least one of body motion parameters and heart rate parameter. The implementation of obtaining the physiological parameters of the user may be obtained through a wearable device of the user. For example, the wearable device is provided with sensors for acquiring the body motion parameters and the heart rate parameters, wherein the sensor for acquiring the body motion parameters may be an acceleration sensor and the sensor for acquiring the heart rate parameters may be a heart rate sensor.

In some embodiments, the body motion parameters may be data for characterizing the body movement of the user. For example, the body motion parameters may be acceleration data of the user's body, which may be acceleration generated during a physical movement of the user. For example, the acceleration data may be the acceleration values generated during the user's hand movements and the time point corresponding to each acceleration value.

S303: determining the sleep state of the user based on the screen state and the physiological parameters.

Wherein, the sleep state includes a sleeping state and an awake state. As an embodiment, the physiological parameters are capable of determining the sleep state of the user. For example, when the user is asleep, the body movement is small and the heart rate exhibits a resting heart rate. If the physiological parameters include body motion parameters and the body motion parameters is acceleration data, the user may be considered to be asleep if the acceleration value is quite low, and if the physiological parameters include heart rate parameters, the user may be considered to be asleep if the heart rate parameters match the user's resting heart rate.

As a result, it is possible to determine whether the user's sleep state is sleeping state or awake state based on the user's physiological parameters, and then, the screen state can reflect the user's intention to operate the target terminal. Specifically, the operating intention can include operating intention and no operating intention. For example, the screen state can include the light-on/light-off state of the screen, and if the screen state is screen-light-on state, the user's intention to operate can be determined as having the intention to operate, and if the screen state is screen-light-off state, the user's intention to operate can be determined as not having the intention to operate. For example, the screen state may include an unlocked/locked state of the screen. If the screen state is unlocked state, the user's operating intention may be determined to be an operating intention, and if the screen state is locked state, the user's operating intention may be determined to be no operating intention. For example, the screen state may include a touch state, and if the screen state is touched state, the user's intention to operate may be determined to be an intention to operate, and if the screen state is untouched state, the user's intention to operate may be determined to be no intention to operate. If there is an intention to operate, the user's sleep state can be considered as sleeping state, and if there is no intention to operate, the user's sleep state can be considered as awake state.

Therefore, the determination result of the user's sleep state can also be obtained according to the screen state, which is noted as a first result, and the determination result of the user's sleep state will be obtained according to the user's physiological parameters, which is noted as a second result, and the sleep state of the user can combining the first result and the second result.

Since the screen state can reflect user's operation of the target terminal, and the operation can further reflect the user's sleep state, the physiological parameters can also reflect the user's sleep state, then, the combination of the screen state and the physiological parameters can make the determination of sleep state more accurate. As illustrated in FIG. 4, an embodiment of the present application provides a method for sleep monitoring, the execution subject of which can be referred to the description of the execution subject of the method illustrated in FIG. 3, it won't be repeated here. Specifically, the method may include: S401 to S406.

S401: obtaining the screen state.

S402: obtaining the physiological parameters of the user.

S403: determining the body state of the user based on the user's physiological parameters.

Wherein, the body state may include a movement state and a heartbeat state, wherein the movement state corresponds to the body motion parameter and the heartbeat state corresponds to the heart rate parameter.

Specifically, the movement state of the user can be determined based on the body motion parameter of the user, which movement state comprises a stationary state and a non-stationary state, in the case of the movement state of the user's body is a stationary state, the user's body has a relatively small action amplitude, and the user's body can be considered to be in a relatively stationary state, in the case of the movement state of the user's body is a non-stationary state, the user's body has a relatively large action amplitude.

As an implementation, the body motion parameter is the acceleration data of the limb of the user, the acceleration data of the user during the detection time period is obtained, if the acceleration data is in a situation where the change in the acceleration data is not large and the acceleration value is lower than the specified acceleration value, then the movement state of the user can be determined as a stationary state, otherwise, the movement state of the user is determined as a non-stationary state. Wherein, the specified acceleration value may be a relatively small value that may be set, for example, based on experience or usage requirements.

In some embodiments, the acceleration data of the user may be collected by a wearable device of the user worn on a limb of the user. For example, the wearable device may be a bracelet or smartwatch, it is in a sleep state when the watch or bracelet is worn with a small acceleration capability value for a sustained relativity long period of time. Specifically, the wearable device may have a three-axis acceleration sensor, then the acceleration data of the user may include the acceleration value of each axis, then the specified acceleration value may include a second threshold value corresponding to each axis, then, determine whether the acceleration value of each axis is less than the specified acceleration value corresponding to that axis, if the acceleration value of each week is less than the specified acceleration value corresponding to that axis, and lasts for a certain time length (e.g., it may be 1 minute), then the movement state of the user's body may be determined as a stationary state, otherwise, the movement state of the user's body is determined as a non-stationary state.

As an implementation, the heartbeat state of the user may comprise a resting state and a non-resting state. After obtaining the heart rate parameter, the real-time heart rate information is determined based on the heart rate parameter, which is used to characterize the speed of the user's heartbeat in real time, it should be noted that the real-time heart rate does not indicate the current heart rate, but may be the heart rate for a period of time up to the current moment. Compare the heart rate parameter with the resting heart rate, if there is no significant increase in the heart rate parameter compared to the resting heart rate, then it may be determined that the heart state of the user is a resting state, otherwise, the heart state of the user is determined to be a non-resting state. As an implementation, the resting heart rate can be the heart rate parameter when the user is in the sleep state during the preset time period, and the resting heart rate is obtained based on the heart rate parameter when the user is in the sleep state, and the preset time period may be 1 to 3 days before the current moment, and the heart rate parameter when the user is in the sleep state during the preset time period is named historical resting heart rate. When the current heart rate is not significantly elevated relative to the historical resting heart rate (a certain threshold range can be set), specifically, it may be to obtain the absolute value of the difference between the current heart rate and the historical resting heart rate, if the absolute value of the difference is less than the resting heartbeat threshold and the length of time that continues to be less than the threshold is higher than the resting time threshold, then it's considered that the heart state of the user is a resting state, otherwise, the heart state of the user is determined to be a non-resting state. Wherein the resting heartbeat threshold is 20 bpm.

Alternatively, in some embodiments, obtaining the physiological parameters of the user is implemented in such a way that the physiological parameters of the user are obtained via a wearable device of the user, and the wearable device of the user having an association with the target terminal. As an implementation, the target terminal may be a mobile terminal, then the mobile terminal has an association with the wearable device. Specifically, the wearable device is bound within the mobile terminal while the user is using the wearable device, i.e., the wearable device is considered to have an association with the mobile terminal if the binding relationship exists between the user device and the wearable device. As another implementation, it is also possible that both the mobile terminal and the wearable device require a login user name to be used properly, and if the current login user name of the mobile terminal is the same as the current login user name of the wearable device, then the wearable device can be considered to be associated with the mobile terminal.

As an implementation, when the wearable device and the mobile terminal are used for monitoring the sleep state of the user, the two need to establish a communication connection, for example, the two are paired via Bluetooth, and then, detect whether the wearable device is in a worn state, if the wearable device and the mobile terminal successfully establish a connection, i.e., successful pairing, and the wearable device is in the worn state, then perform S401 and subsequent steps .

S404: determining whether the body state satisfies preset characteristic conditions.

Wherein, the preset characteristic conditions may be a body state feature of the user in the sleep state.

As an implementation, the body state is a movement state, and the implementation of determining whether the body state satisfies the preset characteristic conditions may be to determine whether the body state is a stationary state, and if it is a stationary state, the body state is determined to satisfy the preset characteristic conditions, otherwise, the body state is determined to do not satisfy the preset characteristic conditions.

As another implementation, the body state is a heartbeat state, and the implementation of determining whether said body state satisfies the preset characteristic conditions may be to determine whether the heartbeat state is a resting state, and if it is a resting state, the body state is determined to satisfy the preset characteristic conditions, otherwise, the body state is determined to do not satisfy the preset characteristic conditions.

As another implementation, the body state is a movement state and a heartbeat state, and the implementation for determining whether the body state satisfies the preset characteristic conditions may be to determine whether the body state is a stationary state and the heartbeat state is a resting state, and if the body state is a stationary state and the heartbeat state is a resting state, the body state is determined to satisfy the preset characteristic conditions, otherwise, the body state is determined to do not satisfy the preset characteristic conditions.

S405: determining the sleep state of the user based on the screen state.

Specifically, the specific implementation of determining the screen-light-on state and the screen-light-off state can be referred to the aforementioned embodiments, and it will not be repeated here. Determine whether the user is in a sleeping state based on the judgement result of the light-on/light-off state of the screen.

Wherein, the judgment result of the light-on/light-off state may comprise the screen state is a screen-light-off state and the screen state is a screen-light-on state.

As an embodiment, the current sleep state of the user may be considered suspected to be a sleeping state if the body state satisfies the preset characteristic conditions, but it is further necessary to determine the current sleep state of the user based on the light-on/light-off state of the screen of the target terminal.

In some embodiments, if the screen state is screen-light-off state, then determined that the sleep state of the user is the sleeping state. Since, in the screen-light-off state, it can be considered that the user is not using the target terminal, and the user can be considered to be asleep if the combined the body state of the user satisfies the preset characteristic conditions.

The light-on/light-off state of the screen of the target terminal can reflect a time period when the user may be asleep, specifically, it is considered that the screen lights on continuously for more than a certain length (e.g., 1 min) is in the awake state, and the screen lights off continuously for more than a certain length is in the sleeping state, therefore, a sleep time period Tp can be determined, and thus, sleep time is a subset of Tp. It is possible to distinguish the scenes of quite-long bed-rest swiping cell phone videos by using this method.

As an implementation, the implementation of determining the sleep state of the user based on the screen state and the physiological parameters may be that the sleep period is determined based on the body motion parameters, heart rate parameters and screen state together, during which the sleep state of the user is the sleeping state and during the time interval outside the sleep period the sleep state of the user is the awake state.

Specifically, determining a first sleep-in time period based on the physiological parameters; determining a second sleep-in time period based on the screen state; determining a target sleep-in time period based on the first sleep-in time period and the second sleep-in time period; and determining that the user's sleep state during the target sleep-in time period is the sleeping state. Specifically, a time interval common to the first sleep-in time period and the second sleep-in time period is used as the target sleep-in time period.

Wherein, the physiological parameters can determine that the user is in the sleep state, for example, by determining the physiological parameters for each moment collected, and determining the body state based on the physiological parameters, if the body state satisfies the aforementioned preset characteristic conditions, it is possible to determine that the user is in the sleep state, thereby being able to obtain a sleep-in time period determined based on the physiological parameters, recorded as the first sleep time period. As an embodiment, if the physiological parameters include body movement parameters and heart rate parameters, then a third sleep-in time period is determined based on the body movement parameters; a fourth sleep-in time period is determined based on the heart rate parameters; and the time interval common to the third sleep-in time period and the fourth sleep-in time period is taken as the first sleep-in time period.

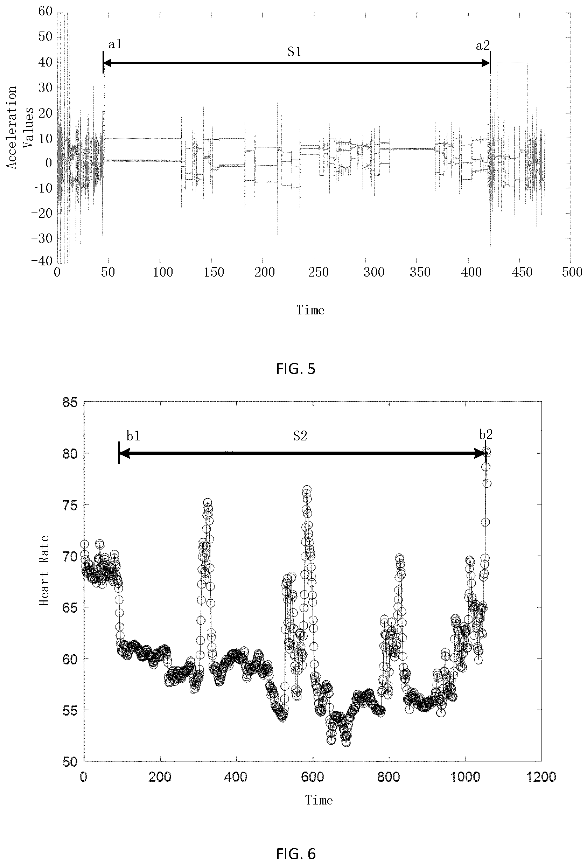

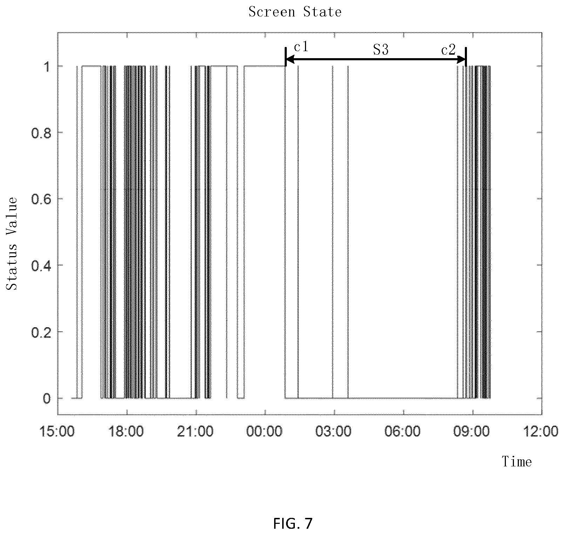

Specifically, as illustrated in FIG. 5, FIG. 6 and FIG. 7, FIG. 5 illustrates a sleep time period S1 of the user determined by acceleration values, and the sleep time period S1 is the third sleep-in time period mentioned above, and it can be seen that within S1, the values of acceleration values are relatively small and stable, wherein a1 is a sleep-in point of the sleep time period S1 and a2 is a wake-up point of the sleep time period S1, i.e., a1 is a first sleep-in point and a2 is a first wake-up point. FIG. 6 illustrates a sleep time period S2 of the user determined by heart rate parameter, and the sleep time period S2 is the fourth sleep-in time period mentioned above, and it can be seen that within S2, the heart rate is relatively small and closer to the resting heart rate of the user, wherein b1 is a sleep-in point of the sleep time period S2 and b2 is a wake-up point of the sleep time period S2, i.e. b1 is a second sleep-in point and b2 is a second wake-up point. FIG. 7 illustrates a sleep time period S3 of the user determined based on screen state, and the sleep time period S3 is the second sleep-in time period mentioned above, wherein the state value is 1 when the screen is in a screen-light-on state and 0 when the screen is in a screen-light-off state, and it can be seen that within S3, the screen is continuously in the screen-light-off state, and in addition, the status value briefly changes to 1 within S3, it may be considered as the screen is briefly lit when the target terminal receives an alert message or a push message. Wherein, c1 is a sleep-in point of the sleep time period S3, and c2 is a wake-up point of the sleep time period S3, i.e., c1 is a third sleep-in point and c2 is a third wake-up point.

As an implementation, the common time interval of the above three sleep-in time periods can be determined, i.e., seeking to intersect of the three time periods, and the common part is a target sleep-in time period, then the target sleep-in time period embodies the sleep-in time period that satisfies the three ways at the same time. Specifically, the latest sleep-in point among the first sleep-in point, the second sleep-in point and the third sleep-in point can be taken as the real fall asleep point of the user, and the earliest wake-up point among the first wake-up point, the second wake-up point and the third wake-up point can be taken as the real wake-up point of the user, then the time period between the real sleep-in point and the real wake-up point is the target sleep-in time period.

In some embodiments, if the body state does not satisfy the preset characteristic conditions, and determining the screen state to be a screen-light-on state, then determine the sleep state of the user to be an awake state.

In some other embodiments, if the screen state is a screen-light-on state and the user's body state satisfies the preset characteristic conditions, the sleep state of the user may be further determined in combination with other information in the case where the screen state is a screen-light-on state.

As an implementation, the unlocked/locked state of the screen of the target terminal can be obtained in the case that the body state of the user satisfies the preset characteristic conditions and the screen state is the screen-light-on state, and if the unlocked/locked state of the screen is the locked state, it can be determined that the sleep state of the user is the sleeping state, and if the unlocked/locked state of the screen is the unlocked state, it can be determined that the sleep state of the user is the awake state.

As another implementation, it is possible to obtain the display content on the screen of the target terminal when the user's body state meets the preset characteristic conditions and the screen state is the screen-light-on state, and if the category of the display content is a given category, then it could determine that the sleep state of the user is the sleeping state, otherwise, it is may determine that the sleep state of the user is the awake state. Wherein, the given category may be a pre-obtained category of content that easily makes the user fall asleep, and as an implementation, the given category may be predetermined by the user, for example, the user marks the content that easily makes the user fall asleep as the given category, for example, marks a dramatic movie as the given category, etc., it may also be a content category of the displayed content on the screen of the target terminal of statistic, in case of that the body state of the user meets the preset characteristic conditions and the screen state is the screen-light-on state during a specified reference time period, thus, multiple content categories for the specified reference time period are get, and determine the given category based on the multiple content categories, for example, the category with the highest frequency of occurrence among the multiple content categories is determined as the given category.

In addition, the category of the displayed content may be determined based on the format of the content, or it may be determined based on the application corresponding to the content. For example, if the application corresponding to the content is a video category, the category of the content displayed on the screen while the application is running is determined to be a video category. Because, the user easily falls asleep while watching the video, it is also possible that when the video playback is completed, the playback interface is automatically exited and other interfaces of the video application are displayed, but, at this time, the user is already in a sleeping state, so determining the category of the displayed content based on the application that plays the displayed content may make the detection of the sleep state more accurate.

Then, the implementation of determining a category of an application may be either the category set by the developer of the application at the time of development or the category set by the user of the application after the application is installed on an electronic device. For example, if the user installs an application on the electronic device, after the installation is completed and the application is accessed, a dialog box is displayed instructing the user to set the category for the application. Then, the specific category to which the application belongs can be set by the user according to his needs. For example, the user can set a social software as audio category, or set it as video category, or set it as social category.

An electronic device has an application installer, such as the Appstore in the IOS system. There is a list of applications in the application installer where users can download applications and update and open them, and the application installer can reality different applications into categories, such as audio, video or games. Thus, the user already knows the category of the application when he installs it with the application installer.

In addition, considering that some applications can play video as well as audio, the category of the application is set to the video category if the application supports the function of video playback, and the category of the application is set to the audio category if it does not support the function of video playback but only supports the function of audio playback. Specifically, it can be determined that whether the application supports the function of video playback by the function description contained in the function description information of the application, for example, the supported playback format, it can also be determined that whether the application supports the function of video playback by detecting whether a video playback module is played within the program module of the application, for example, a certain video playback codec algorithm, etc.

Further, if some applications have diverse functions, it is necessary to determine the category of the application based on the specific operation behavior of the application. For example, if some applications can play video and audio, such as some video playback software, can play pure audio files, but also can play video, the application category can be determined based on the usage records of the application, that is, according to the usage records of the application of a certain time period to determine whether the user prefer to play video or audio with the application.

Specifically, the operation behavior data of all users of the application during the predetermined time period is obtained, wherein, all users means all users who have installed the application, then the operation behavior data can be obtained from the server corresponding to the application, that is, the user logs into the application with the user account corresponding to the user when using the application, and the operation behavior data corresponding to the user account will be sent to the server corresponding to the application, and the server stores the obtained operation behavior data corresponding to the user account. In some embodiments, the electronic device sends an operation behavior query request for the application to the server corresponding to the application, and the server sends all the operation behavior data of the user within a certain preset time period to the electronic device.

The operation behavior data includes the name and time of the audio files played and the name and time of the video files played, and by analyzing the operation behavior data, it is possible to determine the number of audio files and the total time played by the application within a predetermined time period, and it could also obtain the number of video files and the total time played by the application, and then, the category of the application is determined based on the percentage of the total playback time of the audio and video files for the predetermined period of time, specifically, obtain the percentage of the total playback time of the audio and video files within the predetermined time period, and for the purpose of description, record the percentage of the total playback time of the audio files within the predetermined time period as the audio playback percentage, and record the percentage of the total playback time of the video files within the predetermined time period as the video playback percentage, if the video playback percentage is greater than the audio playback percentage, then the category of the application is set to video category, and if the audio playback percentage is greater than the video playback percentage, then the category of the application is set to audio category. For example, if the preset time period is 30 days, i.e. 720 hours, and the total playback time of audio files is 200 hours, then the audio playback percentage is 27.8%, and the total playback time of video files is 330 hours, then the video playback percentage is 45.8%, then the video playback percentage is greater than the audio playback percentage, then the category of the application is set to video category.

As another embodiment, it may also determine the sleep state of the user based on real-time heart rate information in the case of the body state of the user satisfies preset characteristic conditions and the screen state is a screen-light-on state. Specifically, if the screen state is determined to be a screen-light-on state, the real-time heart rate information of the user is obtained based on the heart rate parameter, and if the real-time heart rate information is less than a first threshold value, then the sleep state of the user is determined to be the sleeping state. If the real-time heart rate information is not less than the first threshold, then the sleep state of the user is determined to be the awake state. Wherein, the first threshold may be the resting heart rate as previously described.

Specifically, if the physiological parameter used in determining the body state is a body motion parameter, it is possible to determine whether the sleep state of the user is an awake state or a sleeping state when the user is suspected of being asleep based on the body motion parameter and when the screen is in screen-light-on, and then combine the real-time heart rate information of the user to determine whether the sleep state of the user is the awake state or the sleeping state when the amplitude of movement is relatively small and the screen is screen-light-on, thereby it is possible to determine the sleep state of the user combining the three elements of the body motion parameters, the screen state and the real-time heart rate information, which can make the results more accurate. If the physiological parameters used to determine the body state include real-time heart rate information, the real-time heart rate information can be obtained again when the screen is light-on, thus avoiding inaccurate detection results when the user's heart rate information is detected to indicate that the user is sleeping and the user turns on the target terminal at that time.

As another implementation, it is considered that when the screen is light-on, the user may be operating the target terminal with a small amplitude of movement and resting heart rate. Therefore, if the screen state is a screen-light-on state, the sleep state of the user could be determined based on the heart rate variability information of the user, specifically, please refer to the subsequent embodiments.

Further, it should be noted that, in the case where the body state of the user meets the preset characteristic conditions and the screen state is a screen-light-off state, in addition to the above-mentioned implementation, which can be used to directly determine the sleep state of the user as sleeping state, it is also possible to determine whether the sleep state of the user is the sleeping state or the awake state in the case where the screen state is the screen-light-off state in combination with the heart rate parameters.

As an implementation, if it is determined that the screen state is the screen-light-off state, then according to the heart rate parameter to obtain the real-time heart rate information of the user, if the real-time heart rate information is greater than a third threshold, then determine the sleep state of the user as the awake state; if the real-time heart rate information is not greater than the third threshold, then determine the sleep state of the user as the sleeping state. Wherein, the third threshold may be the same as the first threshold, both of which may be the resting heart rate as described above. Thus, considering that the user is not asleep even though the user is not looking at the screen when the amplitude of body movement is relatively small and the screen is light-off, it is then possible to further determine whether the user is in the sleep state by real-time heart rate information.

As an implementation, if the screen state is determined to be the screen-light-off state, the heart rate variability information of the user is obtained based on the heart rate parameters, and the sleep state of the user is determined based on the heart rate variability information, specifically, see subsequent implementations.

S406: determining the sleep state of the user as the awake state.

If the body state of the user does not meet the preset characteristic conditions, the user's sleep state is determined to be the awake state. It should be noted that the steps not described in detail in the above method can be referred to the preceding embodiments and will not be repeated here.

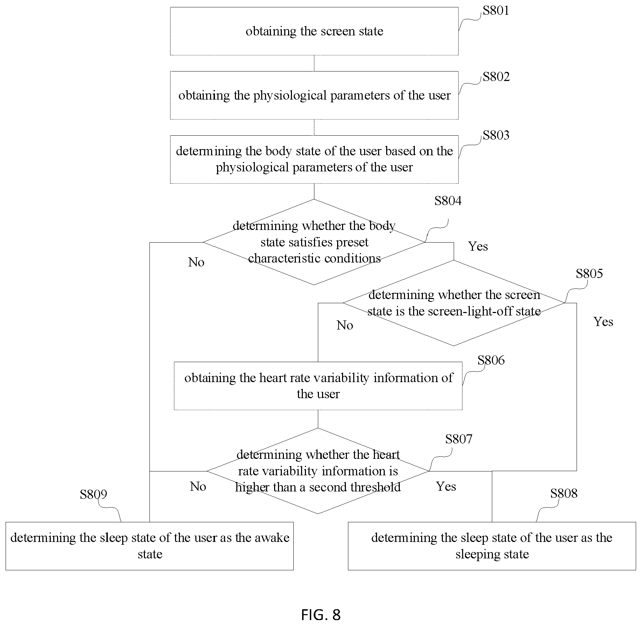

As illustrated in FIG. 8, the present application embodiment provides a method for sleep monitoring, the execution subject of which could be described with reference to the preceding description for the execution subject of the method illustrated in FIG. 3 and FIG. 4, and will not be repeated here. In this application embodiment, the method provided by this application embodiment is described as an example where the target terminal is a mobile terminal, and specifically, the method may include: S801 to S809.

S801: obtaining the screen state.

S802: obtaining the physiological parameters of the user.

S803: determining the body state of the user based on the physiological parameters of the user.

S804: determining whether the body state satisfies preset characteristic conditions.

As an implementation, it may be determined whether the body state of the user satisfies the preset characteristic condition based on either of the acceleration data and the heart rate parameter of the user. Specific implementation paradigms may be referred to the preceding embodiments.

As an implementation, it can be first determined the current sleep state of the user as sleep state to be confirmed in the case of that the body state satisfies the preset characteristic condition, i.e., the user is currently in a state of suspected asleep. To further determine a more realistic sleep state of the user, it can be combined with the screen-light-on/screen-light-off state to determine that,

S805: determining whether the screen state is the screen-light-off state.

If the screen state is the screen-light-off state, it means that the current user's body state meets the user's body state when sleeping, and, the screen is in the screen-light-off state, and since the possibility of the user using the mobile terminal in the screen-light-off state is very small, it can be considered that the user currently has a great possibility of falling asleep, i.e., S808 is executed.

In addition, considering that when the user is lying in bed, he may just listen to music but not fall asleep, but at this time the body movement is relatively small and the heart rate is stable, and since the music playing software can run in the background of the mobile terminal, it is still possible for the mobile terminal to turn off the screen when running the music playing software, so at this time, if according to step S805, the result obtained should be that the sleep state of the user is the sleeping state, thus it will lead to error.

Therefore, it is possible to determine whether the mobile terminal currently has an audio playback-type application running when the judgment result of S805 is the screen-light-off state, specifically, it can be to determine whether there is an audio playback-type application running in the background, and if there is not, then S808 is executed, i.e., it is determined that the sleep state of the user is the sleeping state. If there is, the surrounding ambient sound is collected by the mobile terminal or wearable device, and it is determined whether the surrounding ambient sound is of specified types of voice. For example, the specified types of voice may be snoring, and if there are the specified types of voice, S808 is executed, i.e., determining that the sleep state of the user is the sleeping state, otherwise, S809 is executed, i.e., determining that the sleep state of the user is the awake state.

S806: obtaining the heart rate variability information of the user.

If the screen state is screen-light-on state, the heart rate variability information of the user is obtained according to the heart rate parameter. And determining the sleep state of the user based on the heart rate variability information.

In addition, considering that the screen may light up briefly if a push message or notification is received when the mobile terminal is in the screen-light-off state, it is not necessary to obtain the heart rate variability information of the user and determine the sleep state of the user based on the heart rate variability information if possible, thus, the screen is in the screen-light-off stage at this time, and the lighting-up of the screen due to the push message or notification is only brief. Therefore, if the screen state is a screen-light-on state, the implementation of obtaining the heart rate variability information of the user may be, if the screen state is the screen-light-on state, to obtain a first length of time that the screen state continues to be in the screen-light-on state; if the first length of time is greater than a first specified threshold, to obtain the heart rate variability information of the user. Wherein, the first specified threshold may be set based on experience, for example, setting a relatively small value, for example, the first specified threshold may be 10 seconds, in addition, the first specified threshold may also be set based on the set notification alert length of the mobile terminal, specifically, this first specified threshold may be greater than or equal to this notification alert length, therefore, if the first time length of the continuously lit screen is less than or equal to the first specified threshold, it can be considered that the current screen is lit temporarily.

Heart rate variability (HRV) information refers to the variability of the heart rate cycle-by-cycle variation or the variability of the heart rate fast or slow. Autonomic nerves in the human body regulate functions in the body that are not under subjective control, including heart rate, respiration, blood pressure and digestion. There are two types of autonomic nerves, the sympathetic nerve for "fight or flight" and the parasympathetic nerve for "relaxation or digestion. HRV, can reflect the working condition of your autonomic nerves. If the user is in the "fight or flight" sympathetic dominant mode, the user's HRV will be lower, i.e., if the sympathetic nerves are more active, the HRV will be lower, i.e., the more active the sympathetic nerves are, the lower the HRV will be. If the user is in the parasympathetic dominant mode of "relaxation or digestion", your HRV will be higher. Therefore, HRV can reflect the sympathetic activity of the user.

Therefore, when the user is in the sleep state, the sympathetic nerve activity is not high and the value of HRV is higher, and when the user is in the awake state, the sympathetic nerve activity is higher even though the body movements are small or the heartbeat is in the resting heartbeat state, and therefore the value of HRV is lower.

S807: determining whether the heart rate variability information is higher than a second threshold.

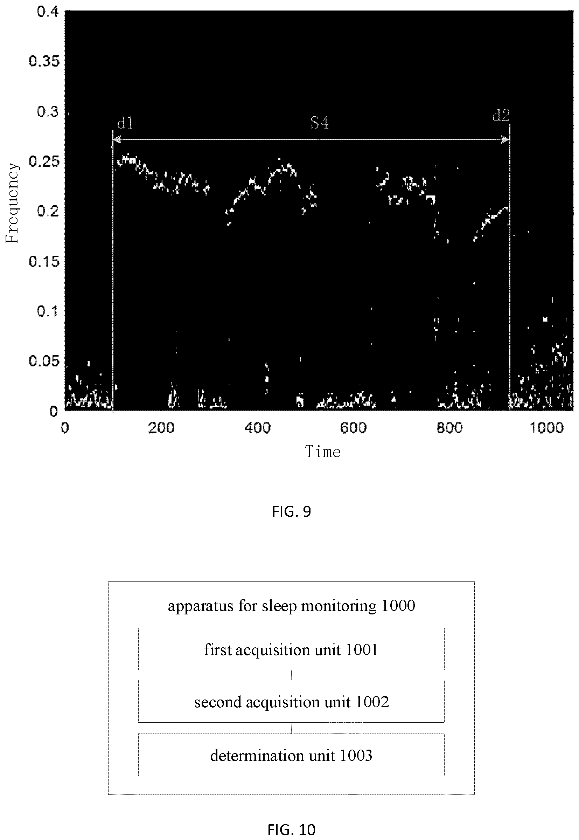

Wherein, the second threshold can be set according to actual needs, for example, it can be a value between 0.2 kHz and 0.3 kHz. As illustrated in FIG. 9, at the position of d1, the value of HRV increases and shows a high frequency state, then it can be determined that the user is currently in a parasympathetic dominant mode, i.e. in a more relaxed state, and therefore, it can be considered that the user's current sleep state is the sleeping state, and at the position of d2, the value of HRV becomes smaller and shows a low frequency state, then it can be determined that the user is currently in a sympathetic dominant mode with sympathetic nerve activity is relatively high and the current sleep state of the user is the awake state.

S808: determining the sleep state of the user as the sleeping state.

In addition, in the case that the body state meets the predetermined characteristic conditions and the screen state is the screen-light-off state, the heart rate variability information of the user is obtained according to the heart rate parameter; if the heart rate variability information is less than the fourth threshold, it is determined that the sleep state of the user is the awake state; if the heart rate variability information is not less than the fourth threshold, it is determined that the sleep state of the user is the sleeping state, wherein the fourth threshold may be the same as the second threshold as previously described, specifically, reference may be made to the aforementioned S806 to S807 and will not be repeated here.

S809: determining the sleep state of the user as the awake state.

As an implementation, if the screen state is the screen-light-on state when the physiological parameters of the user meet the preset characteristic conditions, the heart rate variability information of the user can be obtained, and the sleep state of the user can be determined based on the heart rate variability information, if the result of the determination is that the sleep state of the user is the sleeping state, then in order to avoid a transient elevation of the HRV and a misjudgment of the sleeping state, it can be determined that the HRV is in an elevated condition for a certain length of time, then determine that the sleep state of the user is the sleeping state.

Specifically, a second length of time for which the heart rate variability information continues above a second threshold is obtained; if the second length of time is greater than a second specified threshold, it is determined that the sleep state of the user is the sleeping state. Wherein, the second specified threshold may be set based on experience, for example, it may be from 10 to 20 minutes.

Further, the aforementioned physiological parameters may include at least one of the data of body motion parameters, heart rate parameters, respiration, blood oxygen, body temperature, etc., and may also include the operation data of the wearable device, wherein the operation data of the wearable device may include the screen state of the wearable device, naming the screen state as the first screen state and naming the screen state of the wearable device as the second screen state.

Thus, the above-described implementation of determining the sleep state of the user based on the screen state and physiological parameters may be changed to determine the sleep state of the user based on the first screen state, the second screen state and physiological parameters, then the above-described implementation of determining whether the screen state is an screen-light-off state or a screen-light-on state may be changed to determine the light-on/light-off state of the first screen state and the second screen state. The above implementation of determining the sleep state of the user to be the sleeping state if the screen state is screen-light-off state can be changed to determine the sleep state of the user to be the sleeping state if both the first screen state and the second screen state are the screen-light-off state. The above implementation of obtaining the user's heart rate variability information if the screen state is a screen-light-on state is changed to obtaining the user's heart rate variability information if both the first screen state and the second screen state are screen-light-on states, the specific implementation can be replaced with reference to the aforementioned implementation and will not be repeated here.

Referring to FIG. 10, which illustrates a structural block diagram of an apparatus for sleep monitoring provided by an embodiment of the present application. The apparatus for sleep monitoring 1000 may include: a first acquisition unit 1001, a second acquisition unit 1002, and a determination unit 1003.

The first acquisition unit 1001 is used to obtain screen state of a target terminal.

The second acquisition unit 1002 is used to obtain physiological parameters of a user, the physiological parameters include at least one of body motion parameters and heart rate parameters.

Further, the second acquisition unit 1002 is further used to obtain physiological parameters of the user through the wearable device of the user, the wearable device of the user having an association with the target terminal.

Determination unit 1003 is used to determine the sleep state of the user based on the screen state and physiological parameters

Further, the determination unit 1003 is further used to determine the body state of the user based on the user's physiological parameters; to determine whether the body state satisfies a preset characteristic conditions, the preset characteristic conditions being the body state of the user in a sleep state; if the preset characteristic conditions are satisfied, to determine whether the screen state is the screen-light-off state or screen-light-on state; to determine whether the user is in the sleeping state based on the result of the determination for the light-on/light-off state of the screen; if the preset characteristic conditions are not satisfied, to determine that the sleep state of the user is the awake state.

Further, the determination unit 1003 is further used to determine that the sleep state of the user is the sleeping state if the screen state is the screen-light-off state.

Further, the determination unit 1003 is further used to obtain the heart rate variability information of the user if the screen state is a screen-light-on state, the heart rate variability information is used to characterize the sympathetic nerve activity of the user; to determine that the sleep state of the user is the sleeping state if the heart rate variability information is above a second threshold; to determine that the sleep state of the user is the awake state if the heart rate variability information is not above the second threshold.

Further, the determination unit 1003 is further used to obtain a first length of time that the screen state has been in the screen-light-on state if the screen state is the screen-light-on state; to obtain the heart rate variability information of the user if the first length of time is greater than a first specified threshold.

Further, the determination unit 1003 is further used to obtain a second length of time that the heart rate variability information remains above a second threshold if the heart rate variability information is above a second threshold; to determine that the sleep state of the user is the sleeping state if the second length of time is greater than a second specified threshold.

It will be clear to those skilled in the field that, for the convenience and brevity of the description, the specific working process of the above described apparatus and module can be referred to the corresponding process in the previous method embodiment and will not be repeated herein.

In the embodiments provided by the present application, the modules are coupled to each other either electrically, mechanically or in other forms.

Alternatively, each functional module in each embodiment of the present application may be integrated in a single processing module, or each module may physically exist separately, or two or more modules may be integrated in a single module. The above integrated modules can be implemented either in the form of hardware or in the form of software functional modules.

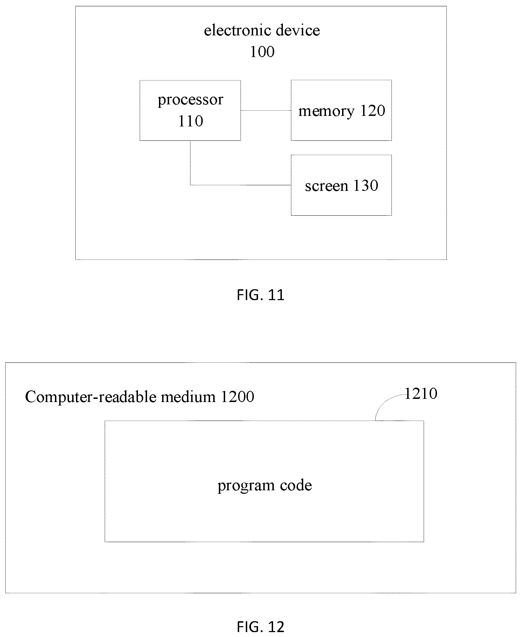

Referring to FIG. 11, which illustrates a block diagram of the structure of an electronic device provided by an embodiment of the present application. The electronic device 100 may be a target terminal or server as described above.

The electronic device 100 of the present application may include one or more of the following components: a processor 110, a memory 120, a screen 130, and one or more applications, wherein the one or more applications may be stored in the memory 120 and configured to be executed by the one or more processors 110, the one or more programs are configured to perform the method as described in the preceding embodiments of method.

The processor 110 may include one or more processing cores. The processors 110 utilize various interfaces and lines to connect various parts within the entire electronic device 100 to perform various functions and process data of the electronic device 100 by running or executing instructions, programs, code sets, or instruction sets stored in the memory 120, and by calling data stored in the memory 120. Optionally, the processor 110 may employ at least one of Digital Signal Processing (DSP), Field-Programmable Gate Array (FPGA), Programmable Logic Array (PLA) to implement at least one of the hardware forms. The processor 110 may integrate one or a combination of one or more of a Central Processing Unit (CPU), a Graphics Processing Unit (GPU), and a modem, etc. Among these, the CPU primarily handles the operating system, user interface, applications, etc.; the GPU is used to take care of rendering and drawing of display content; and the modem is used to handle wireless communication. It can be appreciated that the above modem can also be implemented without being integrated into the processor 110 and through a separate communication chip.

The memory 120 may include Random Access Memory (RAM), or it may include Read-Only Memory. Memory 120 may be used to store instructions, programs, code, code sets, or instruction sets. Memory 120 may include a program storage area and a data storage area, wherein the program storage area may store instructions for implementing an operating system, instructions for implementing at least one function (e.g., a touch function, a sound playback function, an image play function, etc.), instructions for implementing each of the method embodiments described below, etc. The data storage area may also store data created by the electronic device 100 in use (e.g., phone book, audio and video data, chat log data), etc.

Referring to FIG. 12, which illustrates a block diagram of the structure of a computer-readable medium provided by an embodiment of the present application. The computer-readable medium 1200 has program code stored in it, and the program code can be called by a processor to execute the method described in the embodiment of method above.

The computer-readable medium 1200 may be an electronic memory such as a flash memory, an EEPROM (electrically erasable programmable read-only memory), an EPROM, a hard disk, or a ROM. Optionally, the computer-readable medium 1200 includes a non-transitory computer-readable storage medium. The computer-readable medium 1200 has storage space for program code 1210 that performs any of the method steps of the method described above. The program code may be read from or written to one or more computer program products. The program code 1210 may be compressed, for example, in an appropriate form.

Finally, it should be noted that the above embodiments are intended only to illustrate the technical solutions of the present application and not to limit them; although the present application is described in detail with reference to the foregoing embodiments, it is understood by a person of ordinary skill in the art that it is still possible to modify the technical solutions described in the foregoing embodiments or to replace some of the technical features thereof with equivalent ones; and these modifications or replacements do not drive the nature of the corresponding technical solutions out of the respective embodiments of the present application. These modifications or substitutions do not drive the essence of the technical solutions away from the spirit and scope of the technical solutions of the embodiments of the present application.

## Claims

1. A method for sleep monitoring, wherein the method is applied to an electronic device, and the method comprising:
acquiring a screen state of a target terminal;
obtaining physiological parameters of a user, the physiological parameters including at least one of body motion parameters and heart rate parameters; and
determining a sleep state of the user based on the screen state and the physiological parameters, the sleep state comprising a sleeping state and an awake state.

2. The method of claim 1, wherein, determining a sleep state of the user based on the screen state and the physiological parameters, comprises:
determining the body state of the user based on the physiological parameters, the body state comprising at least one of a movement state and a heartbeat state;
determining whether the body state satisfies a preset characteristic condition, the preset characteristic condition being a characteristic of a body state corresponding to the sleep state;
determining the sleep state of the user based on the screen state if the body state satisfies the preset characteristic condition; and
determining the sleep state of the user to be the awake state if the body state does not satisfy the preset characteristic condition.

3. The method of claim 2, wherein determining the sleep state of the user based on the screen state, comprises:
determining the sleep state of the user to be the sleeping state if the screen state is determined to be a screen-light-off state; and
determining the sleep state of the user to be the awake state if the screen state is determined to be a screen-light-on state.

4. The method of claim 2, wherein the physiological parameters comprising heart rate parameters, determining the sleep state of the user based on the screen state, comprises:
determining the sleep state of the user to be the sleeping state if the screen state is determined to be a screen-light-off state;
obtaining real-time heart rate information of the user based on the heart rate parameters if the screen state is determined to be a screen-light-on state;
determining the sleep state of the user to be the sleeping state if the real-time heart rate information is less than a first threshold; and
determining the sleep state of the user to be the awake state if the real-time heart rate information is not less than the first threshold.

5. The method of claim 2, wherein the physiological parameters comprising heart rate parameters, determining the sleep state of the user based on the screen state, comprises:
determining the sleep state of the user to be the sleeping state if the screen state is determined to be a screen-light-off state;
obtaining the heart rate variability information of the user based on the heart rate parameters if the screen state is determined to be a screen-light-on state;
determining the sleep state of the user to be the sleeping state if the heart rate variability information is greater than a second threshold; and
determining the sleep state of the user to be the awake state if the heart rate variability information is not greater than the second threshold.

6. The method of claim 2, wherein the physiological parameters comprising heart rate parameters, determining the sleep state of the user based on the screen state, comprises:
determining the sleep state of the user to be the awake state if the screen state is determined to be a screen-light-on state;
obtaining real-time heart rate information of the user based on the heart rate parameters if the screen state is determined to be a screen-light-off state;
determining the sleep state of the user to be the awake state if the real-time heart rate information is greater than a third threshold; and
determining the sleep state of the user to be the sleeping state if the real-time heart rate information is not greater than the third threshold.

7. The method of claim 2, wherein the physiological parameters comprising heart rate parameters, determining the sleep state of the user based on the screen state, comprises:
determining the sleep state of the user to be the awake state if the screen state is determined to be a screen-light-on state;
obtaining the heart rate variability information of the user based on the heart rate parameters if the screen state is determined to be a screen-light-off state;
determining the sleep state of the user to be the awake state if the heart rate variability information is less than a fourth threshold; and
determining the sleep state of the user to be the sleeping state if the heart rate variability information is not less than the fourth threshold.

8. The method of claim 2, wherein determining the sleep state of the user based on the screen state, comprises:
determining the sleep state of the user to be the sleeping state if the screen state is an untouched state; and
determining the sleep state of the user to be the awake state if the screen state is a touched state.

9. The method of claims 2, wherein determining the sleep state of the user based on the screen state, comprises:
determining the sleep state of the user to be the sleeping state if the screen state is a locked state; and
determining the sleep state of the user to be the awake state if the screen state is an unlocked state.

10. The method of any one of claims 2 to 9, wherein the body state comprising a movement state, determining whether the body state satisfies a preset characteristic condition, comprises:
determining whether the movement state is a stationary state;
determining that the body state satisfies the preset characteristic condition if the movement state is the stationary state; and
determining that the body state does not satisfy the preset characteristic condition if the movement state is a non-stationary state.

11. The method of any one of claims 2 to 9, wherein the body state comprising a heartbeat state, determining whether the body state satisfies a preset characteristic condition, comprises:
determining whether the heartbeat state is a resting state;
determining that the body state satisfies the preset characteristic condition if the heartbeat state is the resting state; and
determining that the body state does not satisfy the preset characteristic condition if the heartbeat state is a non-resting state.

12. The method of any one of claims 2 to 9, wherein the body state comprising a movement state and a resting state, determining whether the body state satisfies a preset characteristic condition, comprises:
determining whether the movement state is a stationary state and the heartbeat state is a resting state;
determining that the body state satisfies the preset characteristic condition if the movement state is the stationary state and the heartbeat state is the resting state; and
otherwise, determining that the body state does not satisfy the preset characteristic condition.

13. The method of any one of claims 2 to 9, wherein determining a sleep state of the user based on the screen state and the physiological parameters, comprises:
determining whether the user is currently in a target sleep time period;
if in, determining the sleep state of the user to be the sleeping state; and
if not in, determining the sleep state of the user to be the awake state.

14. The method of claim 13, wherein before determining whether the user is currently in a target sleep time period, further comprises:
determining a first sleep-in time period based on the physiological parameters, and determining a second sleep-in time period based on the screen state; and
determining the target sleep-in time period based on the first sleep-in time period and the second sleep-in time period.

15. The method of claims 14, wherein determining a first sleep-in time period based on the physiological parameters, comprises:
determining a third sleep-in time period based on the body motion parameters;
determining a fourth sleep-in time period based on the heart rate parameters;
obtaining a time interval common to the third sleep-in time period and the fourth sleep-in time period; and
using the time interval as the first sleep-in time period.

16. The method of any one of claims 4 to 9, wherein the screen state is determined to be a screen-light-on state, comprises:
determining the screen state to be the screen-light-on state if the screen state is continuously in the screen-light-on state for a period longer than a fifth threshold.

17. The method of any one of claims 1 to 9, wherein acquiring a screen state of a target terminal, comprises:
acquiring the screen state of the target terminal associated with the user, the target terminal comprising at least one of a mobile terminal, a wearable device and an AV playback device;
obtaining physiological parameters of a user, comprises:
obtaining the physiological parameters of the user via a wearable device worn by the user, wherein, there is an association between the target terminal and the wearable device.

18. An apparatus for sleep monitoring, wherein the apparatus is applied in an electronic device, and the apparatus comprises:
a first acquisition unit, configured to obtain a screen state of a target terminal;
a second acquisition unit, configured to obtain physiological parameters of a user, the physiological parameters comprising at least one of body motion parameters and heart rate parameters; and
a determination unit, configured to determine a sleep state of the user based on the screen state and the physiological parameters, the sleep state comprising a sleeping state and an awake state.

19. An electronic device, wherein the electronic device comprises:
one or more processors;
a memory;
one or more applications, wherein the one or more applications are stored in the memory and configured to be executed by the one or more processors, the one or more applications are configured to perform the method of any one of claims 1-17.

20. A computer-readable medium, storing a program code executable by a processor, wherein the program code when executed by the processor cause the processor to perform the method of any one of claims 1 to 17.
